# EUROPÄISCHE PATENTSCHRIFT

(11) **EP 1 763 340 B1**
(45) Veröffentlichungstag und Bekanntmachung des Hinweises auf die Patenterteilung: **02.03.2011**
(21) Anmeldenummer: 05770157.5
(22) Anmeldetag: 22.06.2005
(51) Int. Cl.: A61K 9/28

(54) **SCHNELLDISPERGIERBARES, FEINTEILIGES, NICHT ZUR ENTMISCHUNG NEIGENDES PULVERFÖRMIGES FILMÜBERZUGSMITTEL BASIEREND AUF POLYVINYLALKOHOL-POLYETHER-PFROPFCOPOLYMEREN GEKENNZEICHNET DURCH BESONDERE PHYSIKALISCHE STABILITÄT UND NIEDRIGE RAUHIGKEIT**
RAPIDLY DISPERSIBLE, FINE-GRAINED, SEPARATION-RESISTANT PULVERULENT FILM COATING AGENT, BASED ON POLYVINYL ALCOHOL-POLYETHER GRAFT COPOLYMERS AND CHARACTERISED BY A PARTICULAR PHYSICAL STABILITY AND A LOW DEGREE OF ROUGHNESS
AGENT D'ENROBAGE PULVERULENT EN FINES PARTICULES, A DISPERSION RAPIDE ET RESISTANT A LA SEPARATION, A BASE DE COPOLYMERES GREFFES POLYVINYLALCOOL-POLYETHER, CARACTERISE PAR UNE STABILITE PHYSIQUE PARTICULIERE ET UN FAIBLE DEGRE DE RUGOSITE

(30) Priorität: 30.06.2004 DE 102004031835
(43) Veröffentlichungstag der Anmeldung: 21.03.2007
(73) Patentinhaber: BASF SE, 67056 Ludwigshafen (DE)
(72) Erfinder: KOLTER, Karl, 67117 Limburgerhof (DE); ANGEL, Maximilian, 67105 Schifferstadt (DE); HABICH, Andreas, 67346 Speyer (DE)
(86) Internationale Anmeldenummer: PCT/EP2005/006718
(87) Internationale Veröffentlichungsnummer: WO 2006/002808

(56) Entgegenhaltungen:
- WO-A-03/070224
- US-B1- 6 413 590
- US-B1- 6 579 953

## Beschreibung

Die vorliegende Erfindung betrifft schnelldispergierbare Filmüberzüge zum Überziehen von pharmazeutischen Darreichungsformen oder Nahrungsergänzungsmitteln, die aus mindestens einem Polyvinylalkohol-Polyether-Pfropfcopolymer (Komponente A), einem Polyvinylpyrrolidon oder Vinylpyrrolidon-Vinylacetat-Copolymeren (Komponente B) organischen oder anorganischen Pigmenten mit einer mittleren Teilchengröße kleiner 8 µm (Komponenten C), einem Tensid mit einem HLB-Wert größer 10 (Komponente D) und weiteren üblichen Coatingbestandteilen bestehen. Ferner betrifft die Erfindung bestimmte Verfahren zur Herstellung von trockenen Coatingprämixen, wobei die Komponenten fest miteinander verbunden sind und keinerlei Entmischung zeigen, und von wässrigen Coatingsuspensionen sowie deren Auftragen auf feste Darreichungsformen.

Feste Darreichungsformen werden aus den verschiedensten Gründen mit einem schnelllöslichen Überzug versehen. So kann beispielsweise das Aussehen, die Unterscheidbarkeit und die Schluckbarkeit verbessert werden, ein bitterer Geschmack überdeckt werden oder die Darreichungsform gegenüber äußeren Einflüssen wie z. B. Feuchtigkeit oder Sauerstoff geschützt werden. Da sich der Filmüberzug schnell in verschiedenen wässrigen Medien u. a. in künstlichem Magen- und Darmsaft lösen soll, muss der wichtigste Bestandteil der Coatingzubereitung ein wasserlösliches, filmbildendes Polymer sein. Zum Überziehen von Tabletten werden als filmbildende Polymere hauptsächlich Hydroxypropylmethylcellulose und Hydroxypropylcellulose eingesetzt, die jedoch gravierende Nachteile aufweisen. So ist die Viskosität dieser Polymere in Wasser sehr hoch und erlaubt nur eine Konzentration bis ca. 10 %, da aufgrund der hohen Viskosität bei höheren Konzentrationen keine feine Zerstäubung in der Sprühdüse mehr möglich ist und der Überzug rauh, inhomogen und unansehnlich wird. Weiterhin sind diese Polymere sehr spröde und erleiden häufig Risse während der Lagerung, insbesondere wenn der Kern durch Feuchtigkeitsaufnahme oder -abgabe sein Volumen verändert.

Polyvinylalkohol ist ebenfalls als Filmbildner bekannt, wird aber wegen verschiedener Nachteile selten eingesetzt. Die Verwendung von polyvinylalkoholhaltigen Zubereitungen, die aus Polyvinylalkohol, Weichmacher und Talkum bestehen, wird in WO 01/04195 beschrieben. Nachteilig bei diesen Zubereitungen sind die langsame Auflösung bei der Herstellung der wässrigen Coatinglösung, die hohe Viskosität, die niedrige Konzentration in der Sprühlösung, die Verwendung von Weichmachern und die langsame Auflösungsgeschwindigkeit des Filmüberzuges, insbesondere nach Lagerung sowie eine Versprödung des Filmüberzuges nach Lagerung einhergehend mit Rissbildungen. Zudem kommt es bei der Versprühung von höherkonzentrierten Polyvinylalkohollösungen (> 8%) zu Fadenbildung an der Sprühdüse.

Die Verwendung von Polyvinylalkohol-Polyether-Pfropfcopolymeren als Überzugsmittel oder Bindemittel in pharmazeutischen Darreichungsformen oder als Verpackungsmaterial oder als Zusatzstoff in kosmetischen, dermatologischen oder hygienischen Zubereitungen ist beispielsweise aus der WO 00/18375 bekannt. So wird beispielsweise eine Rezeptur für ein Filmüberzugsmittel beschrieben, welches aus einem Polyvinylalkohol-Polyether-Pfropfcopolymer und den üblichen Coatingbestandteilen zum Färben und Decken, nämlich Eisenoxid, Talkum und Titandioxid besteht. Ein so gearteter Überzug ist zwar flexibel, jedoch verhältnismäßig weich und zeigt, wenn Scherkräfte auf ihn einwirken, Abriebserscheinungen. Dies spielt besonders eine Rolle bei sehr großen Coatingansätzen, weil dann der hohe Druck bedingt durch die hohe Schüttung der Tabletten in Verbindung mit der rollierenden Bewegung der Tabletten in der Trommel entsprechend hohe Scherkräfte erzeugt. Da viele Arzneistoffe und auch einige Hilfsstoffe sehr lipophil sind, haften die Überzüge häufig schlecht auf der Tablettenoberfläche. Darüber hinaus sind Glätte und Glanz von solchen Coatingzubereitungen nicht zufriedenstellend.

In der WO 03/070224 werden Überzüge beschrieben, die aus Polyvinylalkohol-Polyether-Pfropfcopolymeren, einer Komponente mit Hydroxy-, Amid- oder Esterfunktionen und weiteren üblichen Coatingbestandteilen bestehen. Dabei wird zunächst ein Prämix der Einsatzstoffe als physikalische Mischung hergestellt und diese anschließend in Wasser dispergiert.Diese Zubereitungen neigen zur Entmischung und weisen keine guten Rauhigkeitswerte auf.

WO03070224 offenbart eine Zusammensetzung für einen Überzug, der folgende Komponenten enthält:
a) 75:25 Polyvinylalkohol-Polyethyleneglykol 6000-Pfropfpolymerzusammensetzung
b) 6:4 Vinylpyrrolidon-Vinylacetat Copolymer - 10 Gew.-%
c) Talkum und Titandioxid
d) Natriumlaurylsulfat (0,5 Ges.-%).
e) weitere Filmüberzugsmittel (wie mikrokristalline Zellulose, Polyethylenoxid-Polypropylenoxid Blockcopolymer).

Die Komponenten werden gemischt und sprühgetrocknet.

Mit US6413590 wird ein Überzug aus Polyvinylalkohol-Polyethylenglykol-Pfropfpolymeren, Polyvinylpyrrolidon und oberflächenaktiven Verbindungen beschrieben.

US6579953 stellt einen Filmüberzug dar, der aus Pfropfpolymeren aus einem Vinylester aliphatischer C1-C24-Carboxylsäuren wie Polyethern besteht, ferner aus anderen Polymeren (wie Polyvinylpyrrolidon), Tensiden und Pigmenten (Talkum, Titandioxid).

Der Erfindung lag die Aufgabe zugrunde, einen Filmüberzug zu entwickeln, der in Pulverform zu keinerlei Entmischung zwischen den einzelnen Bestandteilen führt insbesondere nicht zwischen Pigmenten und Polymeren, der ausgezeichnet fließfähig ist, der sehr einfach und schnell in Wasser aufzulösen bzw. zu ist, wodurch sich eine sehr kurze Herstellungszeit der Sprühzubereitung ergibt, der in hohen Polymer und Feststoffkonzentrationen und mit hoher Sprührate zu versprühen ist, ohne dass die Sprühdüse verblockt, der sehr gut auf der Oberfläche spreitet, der flexibel ist und keinerlei Rissbildung während der Lagerung aufweist, der nicht klebrig ist, der auf allen Oberflächen gut haftet, der ausgezeichnete Glätte und Glanz aufweist, der gegenüber mechanischer Belastung sehr stabil ist und sich sehr schnell auflöst.

Demgemäß wurden pulverförmige Filmüberzugsmittel, bestehend aus
a) 40 - 90 Gew.-% eines Polyvinylalkohol-Polyether-Pfropfcopolymeren (Komponente A),
b) 1 - 20 Gew.-% eines Polyvinylpyrrolidons oder eines Vinylpyrrolidon-Vinylacetat-Copolymeren(Komponente B)
c) 10 - 60 Gew.%organischen oder anorganischen Pigmenten mit einer mittleren Teilchengrösse kleiner 8µm (Komponente C)
d) 0,5 -15 Gew.-% eines Tensids mit einem HLB-Wert größer 10 (Komponente D) sowie
e) 0 - 30 Gew.-% weiteren üblichen Coatingbestandteilen (Komponenten E)
gefunden, in denen die Komponente C in eine kohärente Polymermatrix eingebettet vorliegt,
wobei sich die Menge der Komponenten A bis E zu 100 Gew.-% addiert.
Unter Polyvinylalkohol-Polyether-Pfropfcopolymeren werden Polymere verstanden, die erhältlich sind durch Polymerisation von
a) mindestens einem Vinylester von aliphatischen C₁-C₂₄-Carbonsäuren bevorzugt Vinylacetat, in Gegenwart von
b) Polyethern der allgemeinen Formel I,

   R¹-O-(R²-O)ₓ₋(R³-O)_{y-}(R⁴⁻O)_{z-}R⁵ I
c) in der die Variablen unabhängig voneinander folgende Bedeutung haben:
   - R¹: Wasserstoff, C₁-C₂₄₋Alkyl, R⁶-C(=O)-, Polyalkoholrest; bevorzugt: R₁=H, CH₃-
   - R⁵: Wasserstoff, C₁₋C₂₄₋Alkyl, R⁶-C(=O)-; bevorzugt: R⁵=H
   - R² bis R₄: -(CH₂)_{2-,} -(CH₂)₃-, -(CH₂)₄₋, -CH₂-CH(CH₃)-, -CH₂₋CH(CH₂-CH₃)-, -CH₂-CHOR⁷⁻CH₂-;
   bevorzugt R₂ bis R₄: -(CH₂)₂-, -CH₂₋CH(CH₃)-ganz besonders bevorzugt R₂ bis R₄: -(CH₂)₂-
   - R₆: C₁-C₂₄-Alkyl;
   - R₇: Wasserstoff, C₁₋C₂₄₋Alkyl, R⁶-C(=O)-;
   - x: 1 bis 5000;
   bevorzugt: x = 10 bis 2000;
   ganz besonders bevorzugt: x = 20 bis 500
   - y: 0 bis 5000;
   bevorzugt: y = 0
   - z: 0 bis 5000;
   bevorzugt: z = 0,
   mit der Maßgabe, dass x ≥ 10 ist, wenn y und z = 0 sind,
und anschließende vollständige oder teilweise Verseifung der Polyvinylestergruppen.
x, y, z:
die Berechnung des Molekulargewichts des Polyethers aus x, y, z erfolgt als Mittelwert, da entsprechende Produkte meist eine breite Molgewichtsverteilung besitzen.

Bevorzugt sind Polyether mit einem mittleren Molgewicht zwischen 400 und 50000 g/mol, besonders bevorzugt 1500 bis 20000 g/mol.

Die Herstellung solcher Pfropfcopolymere ist an sich bekannt.

DE 1 077 430 beschreibt ein Verfahren zur Herstellung von Pfropfpolymerisaten von Vinylestern auf Polyalkylenglykole.

DE 1 094 457 und DE 1 081 229 beschreiben Verfahren zur Herstellung von Pfropfpolymerisaten von Polyvinylalkohol auf Polyalkylenglykolen durch Verseifung der Vinylester und deren Verwendung als Schutzkolloide, wasserlösliche Verpackungsfolien, als Schlichte- und Appreturmittel für Textilien und in der Kosmetik.

Bevorzugt sind Polymere mit einem Verseifungsgrad der
Polyvinylestergruppen von > 70 mol-%,
besonders bevorzugt > 80 mol% und
ganz besonders bevorzugt von > 85 mol%.

Besonders bevorzugt ist ein Polyvinylalkohol-Polyether-Pfropfcopolymer bei dem
a) Vinylacetat als zu pfropfendes Monomer verwendet wurde,
b) die Variablen folgende Bedeutung haben:
   R¹= H
   R² - R⁴ = -(CH₂)₂₋
   R₅ = H
   x = 20 bis 500
   y = 0
   z = 0
   und somit ein Polyethylenglykol mit einem mittleren Molekulargewicht von 6000 repräsentieren
c) der Verseifungsgrad der Estergruppen > 85 mol% liegt und
d) das Massenverhältnis der Molekülteile Polyvinylalkohol / Polyethylenglykol 6000 bei 75:25 liegt.

Weiterhin enthalten die Filmüberzugsmittel als Komponenten B ein Polyvinylpyrrolidon oder ein Vinylpyrrolidon-Vinylacetat-Copolymer. Der K-Wert nach Fikentscher, gemessen in Wasser, dieser Polymeren liegt zwischen 10 und 100 vorzugsweise zwischen 12 und 60 und besonders bevorzugt zwischen 20 und 50.

Weiterhin enthalten die Filmüberzugsmittel als Komponenten C organische oder anorganische Pigmente mit einer mittleren Teilchengrösse kleiner 8 µm, bevorzugt < 6 µm, besonders bevorzugt < 4 µm. Die untere Grenze der mittleren Teilchengröße liegt im allgemeinen bei 0,5 µm.

Als Pigmente werden farbgebende oder weiße Substanzen bezeichnet, die im Anwendungsmedium nicht löslich sind.

Als anorganische Pigmente eignen sich Aluminiumsilikate, Magnesiumsilikate, Magnesium-aluminiumsilikate, Eisenoxid, Titandioxid, Zinkoxid, Kieselsäure, oder Calciumhydrogenphosphat. Von den Aluminiumsilikaten kommt vor allem Kaolin in Betracht. Von den Magnesiumsilikaten hat vor allem Talkum Bedeutung.

Bevorzugte Pigmente sind Eisenoxid und Weißpigmente ausgewählt aus der Gruppe bestehend aus Titandioxid, Talkum und Kaolin.

Als organische Pigmente eignen sich organische Farblacke oder Mischungen davon. Als organische Farblacke können z.B. verwendet werden: Chinolingelblack, Tartrazinlack, Gelborangelack, FD&C Gelb-Aluminiumlack, Cochenillerotlack, Erythrosinlack, Azorubinlack, Indigotinlack, Betacarotin.

Als Komponenten D werden Tenside mit einem HLB-Wert (Hydrophilic Lipophilic Balance; Vgl. Fiedler, Lexikon der Hilfsstoffe, Editio Cantor Verlag Aulendorf, 5.Auflage (2002), Seite 115-121) größer 10 eingesetzt.

Besonders geeignet sind Alkalisalze von C8-C30-Fettsäuren , C8-C30-.Alkylsulfonaten, C8-C30-Alkylsulfaten, C8-C30-Alkylarylsulfonaten oder Dioctylsulfosuccinat, Ethoxylate von C8-C30-Fettsäuren, C8-C30-Fettalkoholen, Fettsäureglyceriden, Sorbitanfettsäureestern, Sorbitanfettalkoholethern oder Phenolen, sowie Polyoxypropylen-Polyoxyethylen-Blockcopolymeren. Beispiele aus den genannten Stoffklassen sind Natriumstearat, Natriumoleat, Natriumlaurylsulfonat, Natriumlaurylsulfat, Polyoxyethylen(9)monostearat, Polyoxyethylen(10)stearylcetylether, Polysorbat 80, Polysorbat 20, ethoxyliertes Ricinusöl (35 EO), ethoxyliertes hydriertes Ricinusöl (40 EO), ethoxylierte 12-Hydroxystearinsäure (15 EO), Poloxamer 188, Poloxamer 408.

Weiterhin können die Filmüberzüge als Komponenten E zusätzliche Hilfsstoffe enthalten, wie sie als Coatingbestandteile üblich sind. Weitere übliche Coatingbestandteile umfassen:
wasserlösliche Farbstoffe, Antiklebemittel, Füllstoffe, Glanzverstärker, Schaumverhinderer, Schutzkolloide, Puffersubstanzen, pH-regulierenden Substanzen, Haftvermittler oder Weichmacher.

Die Herstellung der erfindungsgemäßen Zubereitungen erfolgt durch intensive Mahlung und Homogenisierung der Pigmente auf einen hohen Feinheitsgrad und durch Trocknung. Für die Mahlung, die in wässriger Lösung vorgenommen wird, werden Mühlen oder stark scherende Dispergiereinheiten eingesetzt. Besonders geeignet sind Kugelmühlen, Korundscheibenmühlen, Rotor-Stator-Einheiten oder Hochdruckhomogenisatoren. Dabei ist es wichtig, dass die Pigmente auf eine mittlere Teilchengröße kleiner 8 µm, vorzugsweise kleiner 6 µm, besonders bevorzugt kleiner 4 µm zerkleinert werden. Durch die Mahlung in wässriger Lösung besonders in Gegenwart von weiteren Bestandteilen der erfindungsgemäßen Zubereitungen, die als Schutzkolloid oder Benetzungshilfsmittel wirken können, wie Polyvinylpyrrolidon, Vinylpyrrolidon-VinylacetatCopolymere oder Tenside mit einem HLB-Wert größer 10, wird eine Reagglomeration verhindert. Durch die anschließende Sprühtrocknung wird der Feinheitsgrad fixiert.

Die Herstellung kann so vorgenommen werden, dass die Komponente C in Wasser auf die geforderte Teilchengröße gemahlen wird, mit den anderen Komponenten und gegebenenfalls Wasser vereinigt wird und zu einem Pulver oder Granulat getrocknet wird. Eine alternative Verfahrensweise für die Herstellung besteht darin, dass die Komponente C in Wasser in Gegenwart von Komponente A, Komponente B, Komponente D oder Komponente E oder Kombinationen hiervon auf die geforderte Teilchengröße gemahlen wird, mit den restlichen Komponenten und gegebenenfalls Wasser vereinigt wird, und zu einem Pulver oder Granulat getrocknet wird.

Es ist auch möglich, dass die Komponente C in Wasser in Gegenwart der Komponenten B und D sowie gegebenenfalls E auf die geforderte Teilchengröße gemahlen wird, mit einer wässrigen Lösung der Komponente A und gegebenenfalls Komponente E und Wasser vereinigt wird und zu einem Pulver oder Granulat getrocknet wird.

Eine besondere Ausführungsform ist dadurch gekennzeichnet, dass die Komponente C in Wasser in Gegenwart von Gesamtmengen oder Teilmengen der Komponenten A, B, D oder E oder Kombinationen hiervon auf die geforderte Teilchengröße gemahlen wird, zu einem Pulver oder Granulat getrocknet wird und mit den Restmengen pulverförmiger Komponente A, B, D und E abgemischt wird und gegebenenfalls kompaktiert, extrudiert, pelletiert oder granuliert wird.

Die Menge an eingesetzten Wasser wird bei den geschilderten Verfahrensweisen so gewählt, dass die erhaltenen wässrigen Dispersionen der erfindungsgemäßen Filmüberzugsmittel Feststoffgehalte von 5 bis 60, bevorzugt 15 bis 50 Gew.-% aufweisen.

Die Trocknung der wässrigen Dispersionen erfolgt vorzugsweise in eine Sprühtrockner, Schaufeltrockner oder Wirbelschichttrockner. In der Regel wird die zu trocknende Dispersion durch Druck zerstäubt und durch warme Luft getrocknet.

Für die Zerstäubung der Suspension bei der Trocknung werden Drücke größer 0.1 MPa, bevorzugt größer 2.0 MPa, besonders bevorzugt größer 8.0 MPa angewendet. Die Zerstäubung kann mittels Einstoffdüsen, Zweistoffdüsen oder mittels rotierender Scheiben erfolgen.

Die Trocknung erfolgt bei Zulufttemperaturen von 50 - 200°C vorzugsweise bei 80 - 180°C.

Die erfindungsgemäßen Filmüberzugsmittel werden als Pulver mit mittleren Teilchengrößen von > 50 µm, bevorzugt > 100 µm, besonders bevorzugt > 150µm erhalten. Die Obergrenze der mittleren Teilchengröße beträgt < 1000 µm.

Die erfindungsgemäßen Filmüberzugsmittel zeichnen sich dadurch aus, dass die Teilchen der Komponente C in eine kohärente Polymermatrix eingebettet sind. Kohärent bedeutet, dass die Matrix eine durchgehende Phase bildet. Die einzelnen Pigmentteilchen sind vollständig von Polymer umgeben beziehungsweise in die Polymermatrix eingebettet. Die Polymermatrix wird von den polymeren Komponenten A und B gebildet. Die weiteren Komponenten D und E sind homogen in dieser Matrix verteilt. Dies entspricht einer sogenannten "festen Dispersion", bei der die Polymermatrix die äußere Phase bildet, und die Pigmentpartikel die innere Phase. Die Pulverpartikel bestehen also aus einer Polymermatrix, in der die Pigmentteilchen dispergiert sind.

Bei der Redispergierung der Zubereitungen in Wasser für das Überziehen von pharmazeutischen Darreichungsformen lösen sich die wasserlöslichen Polymere und die weiteren wasserlöslichen Bestandteile auf und der ursprüngliche Feinheitsgrad wird wieder erreicht. In dieser wässrigen Dispersion des Filmüberzugsmittels bildet dann die wässrige Lösung die äußere Phase und die Pigmentpartikel die innere Phase, wobei die Teilchengröße < 8 µm der Pigmentpartikel erhalten bleibt.

Dadurch unterscheiden sich die erfindungsgemäßen Zubereitungen erheblich von den bisher üblichen, bei denen die Bestandteile separat nebeneinander vorliegen und sich trennen bzw. reagglomerieren können.

Die erfindungsgemäßen Zubereitungen sind in trockener Form extrem stabil gegen Vibrationen und Belastungen wie sie üblicherweise beim Feststoffhandling z.B. Schütten, Rütteln, Einfüllen, Transportieren, auftreten. Es erfolgt keinerlei Trennung der einzelnen Bestandteile, also keine Entmischung. Die einzelnen Komponenten können nicht durch Siebung oder Sichtung getrennt werden. Somit ist die Zubereitung immer homogen und beim Einwiegen werden immer Mengen mit gleichen Verhältnissen eingewogen.

Die erfindungsgemäßen Filmüberzugsmittel können zunächst als weiße Zubereitung hergestellt werden, denen später weitere pulverförmige farbgebende Komponenten zugesetzt werden können. Die positiven Eigenschaften der Filmüberzüge bleiben auch bei dieser Vorgehensweise erhalten.

Diese Formulierungen können gegebenenfalls kompaktiert, extrudiert, pelletiert oder granuliert werden.

Für die Anwendung werden die erfindungsgemäßen Zubereitungen in Wasser eingerührt, gegebenenfalls mit weiteren Zusätzen insbesondere farbgebenden Zusätzen versetzt wird und mittels einer geeigneten Sprüheinrichtung auf das Substrat aufgebracht wird, wobei durch Zufuhr von erwärmter Luft der Filmüberzug sukzessive getrocknet wird. Für diese Redispergierung werden keine hochscherenden Rührwerkzeuge benötigt, sondern lediglich einfache langsam drehende Rührer. Die Redispergierzeit ist extrem kurz und es wird durch die niedrige Scherung keine Luft eingearbeitet, so dass kein Schaum entsteht. In der Regel ist die Redispergierung nach spätestens 10 min, häufig schon nach 5 min abgeschlossen.

Aufgrund der Feinheit der Pigmente und keiner Reagglomerationstendenz sind diese Dispersionen sehr sedimentationsstabil. Auch bei Unterbrechungen des Rührens erfolgt keine Trennung der Bestandteile. Dies ist überraschend, da die Viskosität der Dispersion sehr niedrig ist. Aufgrund dieser Stabilität wird eine Verblockung der Sprühdüse oder zuführender Schläuche, die bei herkömmlichen Zubereitungen in relativ kurzen Abständen vorkommen, vermieden. Durch die Sedimentationsstabilität setzt sich keinerlei Produkt an oder in der Zerstäubungseinheit ab. Zudem ermöglicht die niedrige Viskosität eine besonders feine Zerstäubung. Vor allem treten keine großen Tropfen auf, die eine negative Auswirkung auf die Glätte, Glanz und Oberflächenstruktur haben. Aufgrund der niedrigen Viskosität können die Dispersionen hochkonzentriert werden, wodurch sich die Sprühzeit stark verkürzt. Die erfindungsgemäße Zubereitungen vereinbaren daher in besonderer Weise Teilchenfeinheit, Viskosität, Redispergiergeschwindigkeit, Sedimentationsstabilität und Versprühbarkeit.

Auf der Oberfläche pharmazeutischer Darreichungsformen ergeben die Zubereitungen Filme außergewöhnlicher Qualität. Durch die Teilchenfeinheit und die gute Zerstäubbarkeit wird eine niedrige Rauhigkeit und guter Glanz erzielt. Trotz eines hohen Pigmentgehaltes liegt die Dehnbarkeit der Filme hoch, wodurch eine Rissbildung auf den Darreichungsformen vermieden wird. Die Reißdehnung überschreitet in der Regel 20 %, vorzugsweise 30 %. Aufgrund der besonderen Zusammensetzung lösen sich die Filme schneller auf als herkömmliche. Dies ist besonders bei Arzneimitteln von Bedeutung, die schnell wirken sollen. Hierbei soll es zu keiner Verzögerung kommen. Üblicherweise lösen sich die Filme in weniger als 2 min, meistens in weniger als 1 min auf.

Die hohe Auflösungsgeschwindigkeit ergibt zudem eine bessere Reinigbarkeit der verwendeten Coatinggerätschaften. Der Reinigungsprozess kann mit einfachen Reinigungsmitteln durchgeführt werden und ist wesentlich schneller als bei Hydroxypropylmethylcellulose- oder Polyvinylalkoholbasierten Coatings. In besonderer Weise ermöglichen die erfindungsgemäßen Zubereitungen sogenannte "Cleaning in place"- Reinigungen.

Die Klebrigkeit der Filme ist in trockenem wie in feuchtem Zustand sehr niedrig. Es finden sich keine Anheftungstendenzen, weder während des Sprühprozesses noch danach bei der Weiterverarbeitung bzw. Lagerung.

Die erfindungsgemäßen Coatingzubereitungen haften besser auf den zu coatenden Darreichungsformen. Dies ermöglicht insbesondere das Coaten von sehr lipophilen Oberflächen, wie Tabletten, die höhere Anteile an lipophilen Wirkstoffen, Wachs oder Fetten enthalten. Übliche Coatingzubereitungen versagen hier, weil die Coatinglösung schlecht spreitet und schlecht haftet. Die guten Spreitungseigenschaften werden insbesondere durch den Einsatz eines Tensides mit einem HLB-Wert größer 10 in Kombination mit den beiden Polymeren erreicht.

Die ausgezeichneten Benetzungseigenschaften zeigen sich auch in der hervorragenden Homogenität des Überzuges. Es finden sich selbst bei dünnen Überzügen und hohen Feststoffkonzentrationen keine sogenannten Pigmentnester, die auf lokale hohe Farbstoffkonzentrationen zurückzuführen sind.

Die Filmüberzüge sind selbst bei sehr hohem Pigment- bzw. Feststoffanteil glatt und glänzend. Die Gravur ist wunderschön nachgebildet. Es kommt zu keinerlei Brückenbildungen oder Feststoffansammlungen in der Gravur. Die überzogenen Darreichungsformen besitzen ein ausgezeichnetes Erscheinungsbild.

Die Sauerstoffdurchlässigkeit der erfindungsgemäßen Zubereitungen ist niedrig, wodurch sauerstoffempfindliche Wirkstoffe im Kern besser geschützt werden können. Durch verminderten oxidativen Abbau wird zudem die Stabilität der eingesetzten Farbstoffe erhöht.

Es sei an dieser Stelle nochmals betont, dass die erfindungsgemäßen Zubereitungen keinerlei Weichmacher bedürfen. Weichmacherfreiheit ist ein enormer Vorteil, weil Weichmacher oft zu Problemen bei der Lagerung von überzogenen Formen führen.

So kann der Weichmacher in den Kern migrieren und den Wirkstoff in seinen physikalischen und chemischen Eigenschaften verändern, der Film versprödet dadurch und neigt zur Rissbildung. Die meisten Weichmacher weisen zudem eine gewisse Flüchtigkeit auf, die ebenfalls zu einer Versprödung führt. Alle diese Nachteile sind bei den erfindungsgemäßen Coatings nicht vorhanden.

Das Aufbringen des Filmüberzugs kann in allen für feste pharmazeutische Darreichungsformen und Nahrungsergänzungsmittel geeigneten Coatingeinrichtungen erfolgen wie z.B. Horizontaltrommelcoatern, Wirbelschichtcoatern, Tauchschwertcoatern, Dragierkesseln.

Für das Zerstäuben der Coatingzubereitung wird vorzugsweise eine Zweistoffdüse verwendet. Die Zulufttemperatur sollte zwischen 30 - 90°C vorzugsweise zwischen 40 - 80°C betragen.

Prinzipiell können alle Kernformen mit gewölbter, konvexer oder konkaver Oberfläche gecoatet werden, unabhängig davon, ob es sich um runde, vieleckige, oblong oder football-shape-Formen handelt.

Durch die niedrigen Viskositäten, die ausgezeichneten Benetzungs- und Spreitungseigenschaften wird eine einzigartige Auskleidung der Gravur erreicht. Es treten keine Brücken- oder Schmiereffekte in der Gravur auf.

Der Kern kann auch ein Subcoating tragen, welches in der Regel aufgebracht wird, um den Wirkstoff besonders zu schützen, z.B. vor Wasser, Sauerstoff, Protonen oder chemischen Stoffen des Überzuges sowie des Magen- und Darminhaltes.

Auf den erfindungsgemäßen Filmüberzug kann auch ein weiterer Filmüberzug aufgetragen werden, der sich in seiner Zusammensetzung unterscheidet. So kann beispielsweise ein farbloser Filmüberzug oder eine besondere Glanzschicht appliziert werden.

Hinsichtlich der Wirkstoffe gibt es für die erfindungsgemäßen Darreichungsformen keine Beschränkungen. Es können Wirkstoffe aller Indikationsgebiete eingesetzt werden, Humanarzneistoffe wie Tierarzneistoffe, Vitamine, Carotinoide, Nutraceuticals, Nahrungsergänzungsstoffe, Mineralstoffe, Mikronährstoffe etc. Die Wirkstoffe können unterschiedliche physikochemische Eigenschaften wie Lipophilie, Löslichkeit, Korngröße, Kornstruktur, Oberfläche etc. besitzen.

Die zu überziehenden Darreichungsformen können als Tabletten, Kapseln oder Extrudate vorliegen.

### Beispiele

Die Prozentangaben beziehen sich, soweit nichts anderes angegeben ist, auf Gew.-%.

Die Angaben zu den Teilchengrößen beziehen sich auf die mittleren Teilchengrößen. Die Bestimmung der Teilchengrößen erfolgte durch Laserbeugung, wobei zur Auswertung der D(4/3)-Wert herangezogen wurde.

Die Bestimmung der Bruchfestigkeit erfolgte auf einem Tablettenprüfautomat der Firma Krämer.

Die Bestimmung der Friabilität erfolgte in einem Erweka-Friabilator 4 min bei 25 Upm.

Die Zerfallszeit wurde nach der Europäischen Pharmakopoeia bestimmt. Die Bestimmung der Freisetzung erfolgte ebenfalls nach der Europäischen Pharmakopoeia.

### Beispiel 1

### Zusammensetzung des Filmüberzug:

| | |
|---|---|
| Polyvinylalkohol-Polyethylenglykol 6000 (75:25) | 60 % |
| Pfropfcopolymer (Verseifungsgrad 94 mol-%) | |
| Vinylpyrrolidon-Vinylacetat 6:4 Copolymer | 10 % |
| (Copolyvidon) | |
| Talkum | 20 % |
| Titandioxid | 9 % |
| Natriumlaurylsulfat | 1 % |

### Herstellung:

Alle Bestandteile wurden in der oben aufgeführten Reihenfolge direkt nacheinander mittels eines Flügelrührers in Wasser eingerührt, so dass eine Feststoffkonzentration von 25 % vorlag. Diese Dispersion wurde mittels einer Skandex-Kugelmühle mit Mahlkörpern 0,6 - 1,25 mm auf eine mittlere Teilchengröße der Pigmentteilchen von 3,5 µm gemahlen und anschließend bei einer Zulufttemperatur von 130°C sprühgetrocknet, so dass ein freifließendes Pulver mit einer Teilchengröße von 105 µm entstand.

### Anwendung

150,0g der Zubereitung wurden mittels eines Blattrührers in 450,0 g Wasser eingerührt. Die Auflösung bzw. Dispergierung war nach 8 min abgeschlossen. Die Sprühsuspension war niedrig viskos und homogen. Die Teilchengröße der Pigmentteilchen nach Redispergierung betrug 3.1 µm.

Die Sprühsuspension wurde in einen Horizontaltrommelcoater (24" Accela-Cota) auf 5 kg Furosemid-Tabletten folgender Zusammensetzung aufgesprüht:

| | |
|---|---|
| Furosemid | 40 mg |
| Ludipress® (BASF AG)¹⁾ | 97,5 mg |
| Copolyvidon | 12,5 mg |
| Mikrokristalline Cellulase ²⁾ | 97,5 mg |
| Magnesiumstearat | 2,5 mg |
| Gesamtgewicht | 250 mg |

| | |
|---|---|
| Durchmesser: 9 mm, gewölbt ¹⁾ Formuliertes Produkt aus 93 Gew.-% Lactose, 3,5 Gew.-% Povidon K30 und 3,5 Gew.-% Crospovidon ²⁾ mittlere Teilchengröße 100 µm | |

### Sprühbedingungen:

| | |
|---|---|
| Zulufttemperatur | 65°C |
| Ablufttemperatur | 33°C |
| Sprührate | 50 g/min |
| Sprühdruck | 0.4 MPa |
| Auftragsmenge | 600 g Sprühdispersion, entsprechend |
| | 150 g Feststoff |
| Sprühzeit | 12 min |

### Filmtabletteneigenschaften:

| | |
|---|---|
| Bruchfestigkeit | 105 N |
| Friabilität | 0 % |
| Zerfallszeit | 3:25 (min:s) |
| Freisetzung | 20 min: 100 % |

Der Überzug war glatt, gleichmäßig und homogen. Die Gravur war schön nachgebildet, ohne Schmiereffekte oder Brückenbildungen. Es war keine Verlängerung der Zerfallszeit oder der Wirkstofffreisetzung gegenüber dem unbeschichteten Tablettenkem Kern festzustellen. Die Bruchfestigkeit war gegenüber dem unbeschichteten Kern um 21 N höher. Während der Stabilitätsprüfung über 12 Monate wurden keine Veränderungen an den Eigenschaften der Filmtabletten festgestellt.

### Beispiel 2

### Zusammensetzung des Filmüberzugs:

| | |
|---|---|
| Polyvinylalkohol-Polyethylenglykol 6000 (75:25) | 61 % |
| Pfropfcopolymer (Verseifungsgrad 94 mol%) | |
| Vinylpyrrolidon-Vinylacetat 6:4 Copolymer (Copolyvidon) | 7 % |
| Kaolin | 16 % |
| Titandioxid | 14 % |
| Natriumlaurylsulfat | 2 % |

### Herstellung:

0,7 kg Copolyvidon wurden in 6,3 kg Wasser gelöst und anschließend 1,6 kg Kaolin und 1,4 kg Titandioxid eingetragen. Diese Grobdispersion wurde in einer Rotor-Stator Dispergiereinheit Typ Coruma 3 h gemahlen, so dass eine mittlere Teilchengröße von 2,0 µm resultierte. Diese Pigmentsuspension wurde in eine Lösung aus 6,1 kg Polyvinylalkohol-Polyethylenglycol-Pfropfcopolymer und 0,2 kg Natriumlaurylsulfat in 17,3 kg Wasser unter Rühren eingetragen. Die Sprühtrocknung erfolgte bei einer Zulufttemperatur von 140°C, wobei der Feinanteil des sprühgetrockneten Pulvers abgetrennt wurde und wieder vor der Sprühdüse eingeblasen wurde, so dass ein Agglomeration und eine gröbere Korngröße resultierte (agglomerierende Sprühtrocknung oder FSD-Technologie). Die Filmüberzugsmittel wurden als freifließende Pulver mit Teilchengrößen von 210 µm erhalten.

### Anwendung

Diese Zubereitung wurde mittels eines Flügelrührers in Wasser eingerührt, so dass eine Sprühzubereitung mit einem Feststoffgehalt von 30 % entstand. Die Auflösung bzw. Dispergierung war nach 6 min abgeschlossen. Die Sprühsuspension war niedrig viskos und homogen. Die Teilchengröße der Pigmentteilchen nach Redispergierung betrug 1.7 µm.

Die Sprühsuspension wurde in einen Horizontaltrommelcoater (24" Accela-Cota) auf 5 kg Ambroxol-HCl-Tabletten aufgesprüht:

### Sprühbedingungen:

| | |
|---|---|
| Zulufttemperatur | 75°C |
| Ablufttemperatur | 37°C |
| Sprührate | 60 g/min |
| Sprühdruck | 0.4 MPa |
| Auftragsmenge | 600g Sprühdispersion, entsprechend 180 g Feststoff |
| Sprühzeit | 10 min |

### Filmtabletteneigenschaften:

| | |
|---|---|
| Bruchfestigkeit | 107 N |
| Friabilität | 0 % |
| Zerfallszeit | 2:23 (min:s) |
| Freisetzung | 20 min: 100 % |

Der Überzug war glatt, gleichmäßig und homogen. Die Gravur war schön nachgebildet, ohne Schmiereffekte oder Brückenbildungen. Es war keine Verlängerung der Zerfallszeit oder der Wirkstofffreisetzung gegenüber dem Kern festzustellen. Die Bruchfestigkeit war gegenüber dem Kern um 25N höher. Während der Stabilitätsprüfung über 12 Monate wurden keine Veränderungen an den Eigenschaften der Filmtabletten festgestellt.

### Beispiel 3

### Zusammensetzung des Filmüberzugs:

| | |
|---|---|
| Polyvinylalkohol-Polyethylenglykol 6000 (75:25) | 40 % |
| Pfropfcopolymer (Verseifungsgrad 94 mol-%) | |
| Polyvinylpyrrolidon K-Wert 30 | 18 % |
| Kaolin | 30 % |
| Titandioxid | 10,5 % |
| Cremophor® RH 40 ¹⁾ | 1,5 % |

ethoxyliertes hydriertes Ricinusöl mit 40 EO-Einheiten

### Herstellung:

1,8 kg Polyvinylpyrrolidon K-Wert 30 wurden in 8,0 kg Wasser gelöst und anschließend 3,0 kg Kaolin und 1,05 kg Titandioxid eingetragen. Diese Grobdispersion wurde in einer Kugelmühle mit Mahlkörpern 0,8 - 1,25 2,5 h gemahlen, so dass eine mittlere Teilchengröße von 2,5 µm resultierte. Diese Pigmentsuspension wurde in eine Lösung aus 4,0 kg Polyvinylalkohol-Polyethylenglycol-Pfropfcopolymer und 0,15 kg Cremophor RH 40 in 22,0 kg Wasser unter Rühren eingetragen. Die Trocknung erfolgte in einem Wirbelschichttrockner bei einer Zulufttemperatur von 90°C. Es wurden freifließende Pulver mit Teilchengrößen von 315 µm erhalten.

### Anwendung

Zur Herstellung der Sprühsuspension wurde die Zubereitung mittels eines Flügelrührers in Wasser eingerührt, so dass eine Sprühzubereitung mit einem Feststoffgehalt von 30 % entstand. Die Auflösung bzw. Dispergierung war nach 7 min abgeschlossen. Die Sprühsuspension war niedrig viskos und homogen. Die Teilchengröße der Pigmentteilchen nach Redispergierung betrug 2,3 µm.

Die Sprühsuspension wurde in einen Horizontaltrommelcoater (24" Accela-Cota) auf 5 kg Coffein-Tabletten folgender Zusammensetzung aufgesprüht:

| | |
|---|---|
| Coffein | 50 mg |
| Ludipress (BASF AG) | 229 mg |
| Mikrokristalline Cellulose ¹⁾ | 40 mg |
| Crospovidon | 10 mg |
| Magnesiumstearat | 1 mg |
| Gesamtgewicht | 330 mg |

| | |
|---|---|
| Durchmesser: 9 mm, gewölbt ¹⁾ mittlere Teilchengröße 100 µm | |

### Sprühbedingungen:

| | |
|---|---|
| Zulufttemperatur | 70°C |
| Ablufttemperatur | 41°C |
| Sprührate | 48 g/min |
| Sprühdruck | 0.45 MPa |
| Auftragsmenge | 600 g Sprühdispersion entspr. 180 g Feststoff |
| Sprühzeit | 12.5 min |

### Filmtabletteneigenschaften:

| | |
|---|---|
| Bruchfestigkeit | 131 N |
| Friabilität | 0 % |
| Zerfallszeit | 2:48 (min:s) |
| Freisetzung | 20 min: 100% |

Der Überzug war glatt, gleichmäßig und homogen. Die Gravur war schön nachgebildet, ohne Schmiereffekte oder Brückenbildungen. Es war keine Verlängerung der Zerfallszeit oder der Wirkstofffreisetzung gegenüber dem Kern festzustellen. Die Bruchfestigkeit war gegenüber dem Kern um 26N höher. Während der Stabilitätsprüfung über 12 Monate wurden keine Veränderungen an den Eigenschaften der Filmtabletten festgestellt.

### Beispiel 4

### Zusammensetzung des Filmüberzugs

| | |
|---|---|
| Polyvinylalkohol-Polyethylenglykol 6000 (75:25) | 53,5 % |
| Pfropfcopolymer (Verseifungsgrad 94 mol%) | |
| Vinylpyrrolidon-Vinylacetat 6:4 Copolymer | 7,5 % |
| (Copolyvidon) | |
| Natriumdihydrogenphosphat | 1 % |
| Titandioxid | 35 % |
| Poloxamer 188 (Lutrol F 68) | 1,25% |
| Polydimethylsiloxan (Simethicon) | 0,25 % |
| Chinolingelblack | 1,5 % |

### Herstellung:

Alle Bestandteile wurden in der oben aufgeführten Reihenfolge direkt nacheinander mittels eines Balkenrührers in Wasser eingerührt, so dass eine Feststoffkonzentration von 30 % vorlag. Diese Dispersion wurde mittels einer Rotor-Stator Dispergiereinheit Typ Coruma 2 h auf eine mittlere Teilchengröße von 1.5 µm gemahlen und anschließend bei einer Zulufttemperatur von 120°C sprühgetrocknet, so dass ein freifließendes Pulver mit Teilchengrößen von 110 µm entstand.

### Anwendung

160,0g der Zubereitung wurden mittels eines Blattrührers in 480,0g Wasser eingerührt. Die Auflösung bzw. Dispergierung war nach 5 min abgeschlossen. Die Sprühsuspension war niedrig viskos und homogen. Die Teilchengröße der Pigmentteilchen nach Redispergierung betrug 1.3 µm.

Die Sprühsuspension wurde in einen Horizontaltrommelcoater (24" Accela-Cota) auf 5 kg Propranolo-HCl-Tabletten folgender Zusammensetzung aufgesprüht:

| | |
|---|---|
| Propranolol-HCl | 50 mg |
| Ludipress (BASF AG) ¹⁾ | 97,5 mg |
| Copolyvidon | 12,5 mg |
| Mikrokristalline Cellulose ²⁾ | 97,5 mg |
| Magnesiumstearat | 2,5 mg |
| Gesamtgewicht | 260 mg |

| | |
|---|---|
| Durchmesser: 9 mm, gewölbt ¹⁾ Formuliertes Produkt aus 93 Gew.-% Lactose, 3,5 Gew.-% Povidon und 3,5 Gew.-% Crospovidon ²⁾ mittlere Teilchengröße 100 mm | |

### Sprühbedingungen:

| | |
|---|---|
| Zulufttemperatur | 65°C |
| Ablufttemperatur | 35°C |
| Sprührate | 45 g / min |
| Sprühdruck | 0.4 MPa |
| Auftragsmenge | 640 g Sprühdispersion, entsprechend |
| | 160 g Feststoff |
| Sprühzeit | 14 min |

### Filmtabletteneigenschaften:

| | |
|---|---|
| Bruchfestigkeit | 110 N |
| Friabilität | 0 % |
| Zerfallszeit | 3:15(min:s) |
| Freisetzung | 20 min: 100 % |

Der Überzug war glatt, gleichmäßig und homogen. Die Gravur war schön nachgebildet, ohne Schmiereffekte oder Brückenbildungen. Es war keine Verlängerung der Zerfallszeit oder der Wirkstofffreisetzung gegenüber dem Kern festzustellen. Die Bruchfestigkeit war gegenüber dem Kern um 26 N höher. Während der Stabilitätsprüfung über 12 Monate wurden keine Veränderungen an den Eigenschaften der Filmtabletten festgestellt.

### Beispiel 5

10,0 kg der weißen Zubereitung aus Beispiel 1 wurden mit 0,5 kg feinst gemahlenem Eisenoxid rot in einem Pflugscharmischer 15 min gemischt und auf einem Gerteis-Walzenkompaktor bei einer Kraft von 3 kN und einer Spaltbreite von 3 mm kompaktiert und das entstandene Kompaktat durch ein Sieb einer Maschenweite 1,0 mm gegeben. Die auf diese Weise hergestellte Zubereitung dispergierte in Wasser innerhalb von 8 min zu einer feinen, stabilen Dispersion.

Diese wurde analog Beispiel 1 auf Tabletten aufgesprüht.

Der Überzug war glatt, gleichmäßig und homogen. Die Gravur war schön nachgebildet, ohne Schmiereffekte oder Brückenbildungen. Es war keine Verlängerung der Zerfallszeit oder der Wirkstofffreisetzung gegenüber dem Kern festzustellen.

### Beispiel 6

150 g der weißen Zubereitung aus Beispiel 2 wurden in 470 g Wasser unter Rühren dispergiert. Sofort anschließend wurde in diese Dispersion ein flüssiger Farbprämix bestehend aus 6 g Indigotinlack, 1 g Poloxamer 188 und 10 g Wasser eingerührt. Nach 10 min Rühren war die Zubereitung sprühfertig und wurde analog Beispiel 2 auf Tabletten aufgesprüht.

Der Überzug war glatt, gleichmäßig und homogen. Die Gravur war schön nachgebildet, ohne Schmiereffekte oder Brückenbildungen. Es war keine Verlängerung der Zerfallszeit oder der Wirkstofffreisetzung gegenüber dem Kern festzustellen. Während der Stabilitätsprüfung über 12 Monate wurden keine Veränderungen an den Eigenschaften der Filmtabletten festgestellt.

## Patentansprüche

1. Schnelldispergierbares, feinteiliges, nicht zur Entmischung neigendes pulverförmiges Filmüberzugsmittel zum Überziehen von pharmazeutischen Darreichungsformen, bestehend aus
a) 40 - 90 Gew.-% eines Polyvinylalkohol-Polyether-Pfropfcopolymeren (Komponente A),
b) 1 - 20 Gew.-% eines Polyvinylpyrrolidons oder eines Vinylpyrrolidon-Vinylacetat-Copolymeren mit einem K-Wert von 10 bis 100 (Komponente B)
c) 10 - 60 Gew.-% organischen oder anorganischen Pigmenten mit einer mittleren Teilchengrösse kleiner 8 µm (Komponente C)
d) 0,5 -15 Gew.-% eines Tensids mit einem HLB-Wert größer 10 (Komponente D) sowie
e) 0 - 30 Gew.-% weiteren üblichen Coatingbestandteilen (Komponenten E), in dem die Teilchen der Komponente C in eine kohärente Polymermatrix eingebettet sind,
wobei sich die Menge der Komponenten A bis E zu 100 Gew.-% addiert.

2. Filmüberzugsmittel nach Anspruch 1, **dadurch gekennzeichnet, dass** Komponente B einen K-Wert zwischen 12 und 60 aufweist.

3. Filmüberzugsmittel nach Anspruch 1 oder 2, **dadurch gekennzeichnet, dass** Komponente B einen K-Wert zwischen 20 und 50 aufweist.

4. Filmüberzugsmittel nach einem der Ansprüche 1 bis 3, **dadurch gekennzeichnet, dass** Komponente B ein Polyvinylpyrrolidon mit einem K-Wert zwischen 25 und 35 oder ein Vinylpyrrolidon-Vinylacetat Copolymer im Verhältnis 6 : 4 mit einem K-Wert zwischen 20 und 40 ist.

5. Filmüberzugsmittel nach einem der Ansprüche 1 bis 4, **dadurch gekennzeichnet, dass** Komponente C eine mittlere Teilchengröße kleiner 6 µm aufweist.

6. Filmüberzugsmittel nach einem der Ansprüche 1 bis 5, **dadurch gekennzeichnet, dass** Komponente C eine mittlere Teilchengröße kleiner 4µm aufweist.

7. Filmüberzugsmittel nach einem der Ansprüche 1 bis 6, **dadurch gekennzeichnet, dass** Komponente C ausgewählt ist aus der Gruppe bestehend aus Aluminiumsilikaten, Magnesiumsilikaten, Magnesium-aluminiumsilikaten, Eisenoxid, Titandioxid, Zinkoxid, Kieselsäure, organischen Farblacken und Mischungen davon.

8. Filmüberzugsmittel nach einem der Ansprüche 1 bis 7, **dadurch gekennzeichnet, dass** Komponente C ausgewählt ist aus der Gruppe bestehend aus Talkum, Kaolin, Titandioxid, Eisenoxid und Mischungen davon.

9. Filmüberzugsmittel nach einem der Ansprüche 1 bis 8, **dadurch gekennzeichnet, dass** Komponente D ausgewählt ist aus der Gruppe bestehend aus Alkalisalzen von Fettsäuren, Alkylsulfonaten, Alkylsulfaten oder Alkylarylsulfonaten, Ethoxylaten von Fettsäuren, Fettalkoholen, Fettsäureglyceriden, Sorbitanfettsäureestern, Sorbitanfettalkoholethern, Phenolen und Polyoxypropylen-Polyoxyethylen-Blockcopolymeren.

10. Filmüberzugsmittel nach einem der Ansprüche 1 bis 9, **dadurch gekennzeichnet, dass** als Komponente D Natriumlaurylsulfat eingesetzt wird.

11. Filmüberzugsmittel nach einem der Ansprüche 1 bis 10, **dadurch gekennzeichnet, dass** Komponente A in einer Konzentration von 50 - 80 Gew.-% enthalten ist

12. Filmüberzugsmittel nach einem der Ansprüche 1 bis 11, **dadurch gekennzeichnet, dass** Komponente B in einer Konzentration von 2 -15 Gew.-% enthalten ist.

13. Filmüberzugsmittel nach einem der Ansprüche 1 bis 12, **dadurch gekennzeichnet, dass** Komponente B in einer Konzentration von 5 -10 Gew.-% enthalten ist.

14. Filmüberzugsmittel nach einem der Ansprüche 1 bis 13, **dadurch gekennzeichnet, dass** Komponente C in einer Konzentration von 15 - 50 Gew.-% enthalten ist.

15. Filmüberzugsmittel nach einem der Ansprüche 1 bis 14 , **dadurch gekennzeichnet, dass** Komponente C in einer Konzentration von 20 - 45 Gew.-% enthalten ist.

16. Filmüberzugsmittel nach einem der Ansprüche 1 bis 15, **dadurch gekennzeichnet, dass** als Komponente C Talkum oder Kaolin in einer Konzentration von 5 - 30 Gew.-% und Titandioxid in einer Konzentration von 10 - 40 Gew.-% eingesetzt werden.

17. Filmüberzugsmittel nach einem der Ansprüche 1 bis 16, **dadurch gekennzeichnet, dass** Komponente D in einer Konzentration von 0,5 -10 Gew.-% enthalten ist.

18. Filmüberzugsmittel nach einem der Ansprüche 1 bis 17, **dadurch gekennzeichnet, dass** Komponente D in einer Konzentration von 1 - 5 Gew.-% enthalten ist.

19. Filmüberzugsmittel nach einem der Ansprüche 1 bis 19, bestehend aus
a) 60 - 65 Gew.-% eines Polyvinylalkohol-Polyether-Pfropfcopolymeren (Komponente A),
b) 5 - 10 Gew.-% eines Vinylpyrrolidon-Vinylacetat-Copolymeren (Komponente B)
c) 14 -18 Gew.-% Talkum oder Kaolin mit einer mittleren Teilchengröße kleiner 8 µm sowie 12 -16 Gew.-% Titandioxid mit einer mittleren Teilchengröße kleiner 8 µm (Komponente C) und
f) 1 - 3 Gew.-% Natriumlaurylsulfat (Komponente D)

20. Filmüberzugsmittel nach einem der Ansprüche 1 bis 19, **dadurch gekennzeichnet, dass** als Komponenten E wasserlösliche Farbstoffe, Antiklebemittel, Füllstoffe, Glanzverstärker, Schaumverhinderer, Schutzkolloide, Puffersubstanzen, pH-regulierenden Substanzen, Haftvermittler oder Weichmacher eingesetzt werden.

21. Filmüberzugsmittel nach einem der Ansprüche 1 bis 20, **dadurch gekennzeichnet, dass** die einzelnen Komponenten innig miteinander verbunden vorliegen und durch Siebung oder Sichtung nicht getrennt werden können.

22. Filmüberzugsmittel nach einem der Ansprüche 1 bis 21, **dadurch gekennzeichnet, dass** nach Redispergierung in Wasser durch einfaches Einrühren die mittlere Teilchengröße des Pigmentteilchens kleiner 8 µm beträgt.

23. Filmüberzugsmittel nach einem der Ansprüche 1 bis 22, **dadurch gekennzeichnet, dass** nach Redispergierung in Wasser durch einfaches Einrühren die mittlere Teilchengröße des Pigmentteilchens kleiner 6 µm beträgt.

24. Filmüberzugsmittel nach einem der Ansprüche 1 bis 23, **dadurch gekennzeichnet, dass** nach Redispergierung in Wasser durch einfaches Einrühren die mittlere Teilchengröße des Pigmentteilchens kleiner 4 µm beträgt.

25. Filmüberzugsmittel nach einem der Ansprüche 1 bis 24, **dadurch gekennzeichnet, dass** die mittlere Teilchengröße des Pulvers größer 50 µm beträgt.

26. Filmüberzugsmittel nach einem der Ansprüche 1 bis 25, **dadurch gekennzeichnet, dass** die mittlere Teilchengröße des Pulvers größer 100 µm beträgt.

27. Filmüberzugsmittel nach einem der Ansprüche 1 bis 26, **dadurch gekennzeichnet, dass** die mittlere Teilchengröße des Pulvers größer 150 µm beträgt.

28. Verfahren zur Herstellung eines Filmüberzugsmittels gemäß einem der Ansprüche 1 bis 27, **dadurch gekennzeichnet, dass** die Komponente C in Wasser auf die geforderte Teilchengröße gemahlen wird, mit den anderen Komponenten und gegebenenfalls Wasser vereinigt wird und die entstandene wässrige Dispersion getrocknet wird.

29. Verfahren zur Herstellung eines Filmüberzugsmittels gemäß einem der Ansprüche 1 bis 27, **dadurch gekennzeichnet, dass** die Komponente C in Wasser in Gegenwart von Komponente A, Komponente B, Komponente D oder Komponente E oder Kombinationen hiervon auf die geforderte Teilchengröße gemahlen wird mit den restlichen Komponenten und gegebenenfalls weiterem Wasser vereinigt wird und die entstandene wässrige Dispersion getrocknet wird.

30. Verfahren zur Herstellung eines Filmüberzugsmittels gemäß einem der Ansprüche 1 bis 27, **dadurch gekennzeichnet, dass** die Komponente C in Wasser in Gegenwart der Komponenten B, und D sowie gegebenenfalls E auf die geforderte Teilchengröße gemahlen wird mit einer wässrigen Lösung der Komponente A und gegebenenfalls weiterem Wasser vereinigt wird und die entstandene wässrige Dispersion getrocknet wird.

31. Verfahren nach einem der Ansprüche 29 bis 30, **dadurch gekennzeichnet, dass** die Komponente C in Wasser in Gegenwart von Gesamtmengen oder Teilmengen der Komponenten A, B, D oder E oder Kombinationen hiervon auf die geforderte Teilchengröße gemahlen wird, zu einem Pulver oder Granulat getrocknet wird und mit den Restmengen pulverförmiger Komponente A, B, D und E abgemischt wird.

32. Verfahren zur Herstellung eines Filmüberzugsmittels gemäß einem der Ansprüche 1 bis 27, **dadurch gekennzeichnet, dass** die Komponente C in Wasser in Gegenwart der Komponenten B, D oder E oder Kombinationen hiervon auf die geforderte Teilchengröße gemahlen wird, die entstandene wässrige Dispersion getrocknet wird und mit pulverförmiger Komponente A abgemischt wird.

33. Verfahren nach einem der Ansprüche 28 bis 32, **dadurch gekennzeichnet, dass** die Trocknung der wässrigen Dispersion durch Sprühtrocknung, Wirbelschichttrocknung, Walzentrocknung oder Sprühgranulierung erfolgt.

34. Verfahren nach Anspruch 33 , **dadurch gekennzeichnet, dass** die Sprühtrocknung bei Zulufttemperaturen von 60 - 200°C erfolgt.

35. Verfahren nach Anspruch 34, **dadurch gekennzeichnet, dass** die Zerstäubung der Suspension bei der Sprühtrocknung bei Drücken größer 0.1 MPa erfolgt.

36. Verfahren nach einem der Ansprüchen 28 bis 35, **dadurch gekennzeichnet, dass** das nach Trocknung der wässrigen Dispersion erhaltenen pulverförmige Produkt mit weiteren pulverförmigen farbgebenden Komponenten abgemischt und gegebenenfalls kompaktiert, extrudiert, pelletiert oder granuliert wird.

37. Verfahren nach einem der Ansprüche 28 bis 36, **dadurch gekennzeichnet, dass** ein nur Weißpigmente enthaltendes pulverförmiges Filmüberzugsmittel mit pulverförmigen farbgebenden Komponenten abgemischt und gegebenenfalls kompaktiert, extrudiert, pelletiert oder granuliert wird.

38. Verfahren zur Herstellung überzogener pharmazeutischer Darreichungsformen, **dadurch gekennzeichnet, dass** ein Filmüberzugsmittel gemäß einem der Ansprüche 1 bis 37 in Wasser eingerührt, gegebenenfalls mit weiteren Zusätzen, insbesondere farbgebenden Zusätzen versetzt wird und mittels einer geeigneten Sprüheinrichtung auf das Substrat aufgebracht wird, wobei durch Zufuhr von erwärmter Luft der Filmüberzug sukzessive getrocknet wird.

39. Feste pharmazeutische Darreichungsformen, überzogen mit einem Filmüberzugsmittel gemäß einem der Ansprüche 1 bis 37.

40. Feste pharmazeutische Darreichungsformen nach Anspruch 39, **dadurch gekennzeichnet, dass** sie als Wirkstoffe Arzneistoffe, Vitamine, Carotinoide, Mineralien, Nahrungsergänzungsmittel oder Spurenelemente enthalten.

41. Feste pharmazeutische Darreichungsformen nach Anspruch 39 oder 40, **dadurch gekennzeichnet, dass** Tabletten, Extrudate oder Kapseln mit einem Filmüberzug versehen werden.

## Claims

1. A rapidly dispersible, fine-particle film-coating composition which is in powder form and is not prone to segregation for coating pharmaceutical dosage forms, consisting of
a) 40 - 90% by weight of a polyvinyl alcohol-polyether graft copolymer (component A),
b) 1 - 20% by weight of a polyvinylpyrrolidone or of a vinylpyrrolidone-vinyl acetate-copolymer with a K value of from 10 to 100 (component B)
c) 10 - 60% by weight of organic or inorganic pigments having an average particle size of less than 8 µm (component C)
d) 0.5 - 15% by weight of a surfactant having an HLB of greater than 10 (component D) and
e) 0 - 30% by weight of further customary coating ingredients (components E),
in which the particles of component C are embedded in a coherent polymer matrix,
where the total amount of components A to E is 100% by weight.

2. The film-coating composition according to claim 1, wherein component B has a K value between 12 and 60.

3. The film-coating composition according to claim 1 or 2, wherein component B has a K value betweem 20 and 50.

4. The film-coating composition according to any of claims 1 to 3, wherein component B is a polyvinylpyrrolidone with a K value between 25 and 35 or a vinylpyrrolidone-vinyl acetate copolymer in the ratio 6 : 4 with a K value between 20 and 40.

5. The film-coating composition according to any of claims 1 to 4, wherein component C has an average particle size of less than 6 µm.

6. The film-coating composition according to any of claims 1 to 5, wherein component C has an average particle size of less than 4 µm.

7. The film-coating composition according to any of claims 1 to 6, wherein component C is selected from the group consisting of aluminum silicates, magnesium silicates, magnesium aluminum silicates, iron oxide, titanium dioxide, zinc oxide, silica, organic lakes and mixtures thereof.

8. The film-coating composition according to any of claims 1 to 7, wherein component C is selected from the group consisting of talc, kaolin, titanium dioxide, iron oxide and mixtures thereof.

9. The film-coating composition according to any of claims 1 to 8, wherein component D is selected from the group consisting of alkali metal salts of fatty acids, alkysulfonates, alkyl sulfates or alkylarylsulfonates, ethoxylates of fatty acids, fatty alcohols, fatty acid glycerides, sorbitan fatty acid esters, sorbitan fatty alcohol ethers, phenols and polyoxypropylene-polyoxyethylene block copolymers.

10. The film-coating composition according to any of claims 1 to 9, wherein sodium lauryl sulfate is employed as component D.

11. The film-coating composition according to any of claims 1 to 10, wherein component A is present in a concentration of 50 - 80% by weight.

12. The film-coating composition according to any of claims 1 to 11, wherein component B is present in a concentration of 2 - 15% by weight.

13. The film-coating composition according to any of claims 1 to 12, wherein component B is present in a concentration of 5 - 10% by weight.

14. The film-coating composition according to any of claims 1 to 13 , wherein component C is present in a concentration of 15 - 50% by weight.

15. The film-coating composition according to any of claims 1 to 14 , wherein component C is present in a concentration of 20 - 45% by weight.

16. The film-coating composition according to any of claims 1 to 15, wherein talc or kaolin in a concentration of 5 - 30% by weight and titanium dioxide in a concentration of 10 - 40% by weight are employed as component C.

17. The film-coating composition according to any of claims 1 to 16, wherein component D is present in a concentration of 0.5 - 10% by weight.

18. The film-coating composition according to any of claims 1 to 17, wherein component D is present in a concentration of 1 - 5% by weight.

19. The film-coating composition according to any of claims 1 to 18, consisting of
a) 60 - 65% by weight of a polyvinyl alcohol-polyether graft copolymer (component A),
b) 5 - 10% by weight of a vinylpyrrolidone-vinyl acetate copolymer (component B)
c) 14 - 18% by weight of talc or kaolin having an average particle size of less than 8 µm, and 12 - 16% by weight of titanium dioxide having an average particle size of less than 8 µm (component C) and
d) 1 - 3% by weight of sodium lauryl sulfate (component D)

20. The film-coating composition according to any of claims 1 to 19, wherein water-soluble colorants, non-stick agents, fillers, gloss improvers, antifoams, protective colloids, buffer substances, pH-regulating substances, bonding agents or plasticizers are employed as components E.

21. The film-coating composition according to any of claims 1 to 20, wherein the individual components are intimately associated with one another and cannot be separated by sieving or sifting.

22. The film-coating composition according to any of claims 1 to 21, wherein the average particle size of the pigment particle is less than 8 µm after redispersion in water by simple stirring in.

23. The film-coating composition according to any of claims 1 to 22, wherein the average particle size of the pigment particle is less than 6 µm after redispersion in water by simple stirring in.

24. The film-coating composition according to any of claims 1 to 23, wherein the average particle size of the pigment particle is less than 4 µm after redispersion in water by simple stirring in.

25. The film-coating composition according to any of claims 1 to 24, wherein the average particle size of the powder is greater than 50 µm.

26. The film-coating composition according to any of claims 1 to 25, wherein the average particle size of the powder is greater than 100 µm.

27. The film-coating composition according to any of claims 1 to 26, wherein the average particle size of the powder is greater than 150 µm.

28. A process for producing a film-coating composition according to any of claims 1 to 27, which comprises grinding component C in water to the required particle size, combining with the other components and, where appropriate, water, and drying the resulting aqueous dispersion.

29. A process for producing a film-coating composition according to any of claims 1 to 27, which comprises grinding component C in water in the presence of component A, component B, component D or component E or combinations thereof to the required particle size combining with the remaining components and, where appropriate, further water, and drying the resulting aqueous dispersion.

30. A process for producing a film-coating composition according to any of claims 1 to 27, which comprises grinding component C in water in the presence of components B and D and, where appropriate, E to the required particle size combining with an aqueous solution of component A and, where appropriate, further water, and drying the resulting aqueous dispersion.

31. A process according to any of claims 29 to 30, which comprises grinding component C in water in the presence of complete quantities or partial quantities of components A, B, D or E or combinations thereof to the required particle size, drying to a powder or granules, and mixing with the remaining quantities of component A, B, D and E in powder form.

32. A process for producing a film-coating composition according to any of claims 1 to 27, which comprises grinding component C in water in the presence of components B, D or E or combinations thereof to the required particle size, drying the resulting aqueous dispersion and mixing with component A in powder form.

33. The process according to any of claims 28 to 32, wherein the drying of the aqueous dispersion takes place by spray drying, fluidized bed drying, drum drying or spray granulation.

34. The process according to claim 33, wherein the spray drying takes place at inlet air temperatures of 60 - 200°C.

35. The process according to claim 34, wherein the atomization of the suspension in the spray drying takes place at pressures greater than 0.1 MPa.

36. The process according to any of claims 28 to 35, wherein the product obtained in powder form after drying of the aqueous dispersion is mixed with further coloring components in powder form and, where appropriate, compacted, extruded, pelleted or granulated.

37. The process according to any of claims 28 to 36, wherein a film-coating composition which is in powder form and comprises only white pigments is mixed with coloring components in powder form and, where appropriate, compacted, extruded, pelleted or granulated.

38. A process for producing coated pharmaceutical dosage forms, which comprises a film-coating composition according to any of claims 1 to 37 being stirred into water, mixed where appropriate with further additions, in particular coloring additions, and applied to the substrate by means of a suitable spraying device, with successive drying of the film coating by feeding in heated air.

39. A solid pharmaceutical dosage form coated with a film-coating composition according to any of claims 1 to 37.

40. The solid pharmaceutical dosage form according to claim 39, which comprises as active ingredients medicinal substances, vitamins, carotenoids, minerals, dietary supplements or trace elements.

41. The solid pharmaceutical dosage form according to claim 39 or 40, wherein tablets, extrudates or capsules are provided with a film coating.

## Revendications

1. Agent de pelliculage pulvérulent finement divisé, à dispersion rapide, n'ayant pas tendance à la démixtion, pour l'enrobage de formes d'administration pharmaceutiques, constitué de
a) 40 - 90 % en poids d'un copolymère greffé poly(alcool vinylique)-polyéther (composant A),
b) 1 - 20 % en poids d'une polyvinylpyrrolidone ou d'un copolymère vinylpyrrolidone/acétate de vinyle ayant un indice K de 10 à 100 (composant B)
c) 10 - 60 % en poids de pigments organiques ou inorganiques ayant une taille moyenne de particule inférieure à 8 µm (composant C)
d) 0,5 - 15 % en poids d'un tensioactif ayant un indice HLB supérieur à 10 (composant D) ainsi que
e) 0 - 30 % en poids d'autres composants d'enrobage usuels (composants E),
dans lequel les particules du composant C sont incluses dans une matrice polymère cohérente,
la somme des quantités des composants A à E étant égale à 100 %.

2. Agent de pelliculage selon la revendication 1, **caractérisé en ce que** le composant B présente un indice K compris entre 12 et 60.

3. Agent de pelliculage selon la revendication 1 ou 2, **caractérisé en ce que** le composant B présente un indice K compris entre 20 et 50.

4. Agent de pelliculage selon l'une quelconque des revendications 1 à 3, **caractérisé en ce que** le composant B est une polyvinylpyrrolidone ayant un indice K compris entre 25 et 35 ou un copolymère vinylpyrrolidone/acétate de vinyle en le rapport 6:4, ayant un indice K compris entre 20 et 40.

5. Agent de pelliculage selon l'une quelconque des revendications 1 à 4, **caractérisé en ce que** le composant C présente une taille de particule inférieure à 6 µm.

6. Agent de pelliculage selon l'une quelconque des revendications 1 à 5, **caractérisé en ce que** le composant C présente une taille de particule inférieure à 4 µm.

7. Agent de pelliculage selon l'une quelconque des revendications 1 à 6, **caractérisé en ce que** le composant C est choisi dans le groupe constitué par les silicates d'aluminium, les silicates de magnésium, les aluminosilicates de magnésium, l'oxyde de fer, le dioxyde de titane, l'oxyde de zinc, l'acide silicique, des laques colorées organiques et des mélanges de ceux-ci.

8. Agent de pelliculage selon l'une quelconque des revendications 1 à 7, **caractérisé en ce que** le composant C est choisi dans le groupe constitué par le talc, le kaolin, le dioxyde de titane, l'oxyde de fer et des mélanges de ceux-ci.

9. Agent de pelliculage selon l'une quelconque des revendications 1 à 8, **caractérisé en ce que** le composant D est choisi dans le groupe constitué par les sels alcalins d'acides gras, les alkylsulfonates, 1 alkylsulfates ou alkylarylsulfonates, les produits d'éthoxylation d'acides gras, les alcools gras, les glycérides d'acides gras, 1 les esters d'acides gras et de sorbitanne, 1 les éthers d'alcools gras et de sorbitanne, 1 les phénols et les copolymères séquencés polyoxypropylène-polyoxyéthylène.

10. Agent de pelliculage selon l'une quelconque des revendications 1 à 9, **caractérisé en ce qu'**on utilise comme composant D le laurylsulfate de sodium.

11. Agent de pelliculage selon l'une quelconque des revendications 1 à 10, **caractérisé en ce que** le composant A est contenu à une concentration de 50 - 80 % en poids.

12. Agent de pelliculage selon l'une quelconque des revendications 1 à 11, **caractérisé en ce que** le composant B est contenu à une concentration de 2 - 15 % en poids.

13. Agent de pelliculage selon l'une quelconque des revendications 1 à 12, **caractérisé en ce que** le composant B est contenu à une concentration de 5 - 10 % en poids.

14. Agent de pelliculage selon l'une quelconque des revendications 1 à 13, **caractérisé en ce que** le composant C est contenu à une concentration de 15 - 50 % en poids.

15. Agent de pelliculage selon l'une quelconque des revendications 1 à 14, **caractérisé en ce que** le composant C est contenu à une concentration de 20 - 45 % en poids.

16. Agent de pelliculage selon l'une quelconque des revendications 1 à 15, **caractérisé en ce qu'**on utilise comme composant C du talc ou du kaolin à une concentration de 5 - 30 % en poids et du dioxyde de titane à une concentration de 10 - 40 % en poids.

17. Agent de pelliculage selon l'une quelconque des revendications 1 à 16, **caractérisé en ce que** le composant D est contenu à une concentration de 0,5 - 10 % en poids.

18. Agent de pelliculage selon l'une quelconque des revendications 1 à 17, **caractérisé en ce que** le composant D est contenu à une concentration de 1 - 5 % en poids.

19. Agent de pelliculage selon l'une quelconque des revendications 1 à 18, constitué de
a) 60 - 65 % en poids d'un copolymère greffé poly(alcool vinylique)-polyéther (composant A),
b) 5 - 10 % en poids d'un copolymère vinylpyrrolidone-acétate de vinyle (composant B)
c) 14 - 18 % en poids de talc ou kaolin ayant une taille moyenne de particule inférieure à 8 µm ainsi que 12 - 16 % en poids de dioxyde de titane ayant une taille moyenne de particule inférieure à 8 µm (composant C) et
d) 1 - 3 % en poids de laurylsulfate de sodium (composant D).

20. Agent de pelliculage selon l'une quelconque des revendications 1 à 19, **caractérisé en ce qu'**on utilise comme composants E des colorants hydrosolubles, des antiadhésifs, des charges, des agents accentuant le brillant, des antimousses, des colloïdes protecteurs, des substances tampon, des substances régulant le pH, des promoteurs d'adhérence ou des plastifiants.

21. Agent de pelliculage selon l'une quelconque des revendications 1 à 20, **caractérisé en ce que** les composants individuels sont présents intimement liés entre eux et ne peuvent pas être séparés par criblage ou tamisage.

22. Agent de pelliculage selon l'une quelconque des revendications 1 à 21, **caractérisé en ce qu'**après redispersion dans l'eau par simple délayage la taille moyenne de particule de la particule de pigment est inférieure à 8 µm.

23. Agent de pelliculage selon l'une quelconque des revendications 1 à 22, **caractérisé en ce qu'**après redispersion dans l'eau par simple délayage la taille moyenne de particule de la particule de pigment est inférieure à 6 µm.

24. Agent de pelliculage selon l'une quelconque des revendications 1 à 23, **caractérisé en ce qu'**après redispersion dans l'eau par simple délayage la taille moyenne de particule de la particule de pigment est inférieure à 4 µm.

25. Agent de pelliculage selon l'une quelconque des revendications 1 à 24, **caractérisé en ce que** la taille moyenne de particule de la poudre est supérieure à 50 µm.

26. Agent de pelliculage selon l'une quelconque des revendications 1 à 25, **caractérisé en ce que** la taille moyenne de particule de la poudre est supérieure à 100 µm.

27. Agent de pelliculage selon l'une quelconque des revendications 1 à 26, **caractérisé en ce que** la taille moyenne de particule de la poudre est supérieure à 150 µm.

28. Procédé pour la préparation d'un agent de pelliculage selon l'une quelconque des revendications 1 à 27, **caractérisé en ce qu'**on broie à la taille de particule requise le composant C dans de l'eau, on le réunit avec les autres composants et éventuellement de l'eau, puis on sèche la dispersion aqueuse résultante.

29. Procédé pour la préparation d'un agent de pelliculage selon l'une quelconque des revendications 1 à 27, **caractérisé en ce qu'**on broie à la taille de particule requise le composant C dans de l'eau en présence du composant A, du composant B, du composant D ou du composant E ou d'associations de ceux-ci, on le réunit avec les composants restants et éventuellement une nouvelle quantité d'eau, puis on sèche la dispersion aqueuse résultante.

30. Procédé pour la préparation d'un agent de pelliculage selon l'une quelconque des revendications 1 à 27, **caractérisé en ce qu'**on broie à la taille de particule requise le composant C dans de l'eau en présence des composants B et D ainsi qu'éventuellement E, on le réunit avec une solution aqueuse du composant A et éventuellement une nouvelle quantité d'eau, puis on sèche la dispersion aqueuse résultante.

31. Procédé selon l'une quelconque des revendications 29 et 30, **caractérisé en ce qu'**on broie à la taille de particule requise le composant C dans de l'eau en présence des quantités totales ou de quantités partielles des composants A, B, D ou E ou d'associations de ceux-ci, on le sèche en une poudre ou un produit granulé et on le mélange avec les quantités restantes des composants pulvérulents A, B, D et E.

32. Procédé pour la préparation d'un agent de pelliculage selon l'une quelconque des revendications 1 à 27, **caractérisé en ce qu'**on broie à la taille de particule requise le composant C dans de l'eau en présence des composants B, D ou E ou d'associations de ceux-ci, on sèche la dispersion aqueuse résultante et on la mélange avec le composant A pulvérulent.

33. Procédé selon l'une quelconque des revendications 28 à 32, **caractérisé en ce que** le séchage de la dispersion aqueuse s'effectue par séchage par atomisation, séchage en lit fluidisé, séchage sur cylindres ou granulation par pulvérisation.

34. Procédé selon la revendication 33, **caractérisé en ce que** le séchage par atomisation s'effectue à des températures d'air entrant de 60 - 200 °C.

35. Procédé selon la revendication 34, **caractérisé en ce que** la pulvérisation de la suspension lors du séchage par atomisation s'effectue sous des pressions supérieures à 0,1 MPa.

36. Procédé selon l'une quelconque des revendications 28 à 35, **caractérisé en ce que** le produit pulvérulent obtenu après séchage de la dispersion aqueuse est mélangé avec d'autres composants colorants pulvérulents et éventuellement compacté, extrudé, mis sous forme de pastilles ou granulé.

37. Procédé selon l'une quelconque des revendications 28 à 36, **caractérisé en ce qu'**un agent de pelliculage pulvérulent contenant seulement des pigments blancs est mélangé avec des composants colorants pulvérulents et éventuellement compacté, extrudé, mis sous forme de pastilles ou granulé.

38. Procédé pour la fabrication de formes d'administration pharmaceutiques enrobées, **caractérisé en ce que** qu'on délaie dans de l'eau un agent de pelliculage selon l'une quelconque des revendications 1 à 37, éventuellement on y ajoute d'autres additifs, en particulier des additifs colorants, et on l'applique sur le substrat au moyen d'un dispositif de pulvérisation approprié, l'enrobage pelliculaire étant successivement séché par introduction d'air chaud.

39. Formes d'administration pharmaceutiques solides, enrobées avec un enrobage pelliculaire selon l'une quelconque des revendications 1 à 37.

40. Formes d'administration pharmaceutiques solides selon la revendication 39, **caractérisées en ce qu'**elles contiennent comme substances actives des médicaments, des vitamines, des caroténoïdes, des minéraux, des compléments nutritionnels ou des oligo-éléments.

41. Formes d'administration pharmaceutiques solides selon la revendication 39 ou 40, **caractérisée en ce que** des comprimés, des extrudats ou des gélules sont munis d'un pelliculage.
